# EUROPEAN PATENT APPLICATION

(11) **EP 2 522 295 A1**
(43) Date of publication of application: **14.11.2012**
(21) Application number: 11425128.3
(22) Date of filing: 10.05.2011
(51) Int. Cl.: A61B 19/00

(54) **Vitual platform for pre-surgery simulation and relative bio-mechanic validation of prothesis surgery of the lumbo-sacral area of the human spine**

(71) Applicant: Loran S.r.l., 70026 Modugno (BA) (IT)
(72) Inventor: Uva, Antonio Emmanuele, 70124 Bari (IT); Solaro, Pierpaolo, 70010 Capurso (BA) (IT); Moramarco, Vincenzo, 70022 Altamura (BA) (IT); Pupilla, Eliana, 70124 Bari (IT); Boffi, Patrizia, 70126 Bari (IT); Lorusso, Nicola, 70010 Cellamare (BA) (IT)
(74) Representative: Bruni, Giovanni

(57) **Abstract**

Virtual platform composed by two modules, a first module for the virtual pre-surgery simulation of a prosthesis surgery of the lumbosacral area of the human spine and a second module for the bio-mechanic validation of the simulated surgery, based on techniques of analysis to the finite elements. The platform comprises a device for navigating in 3D environment with 6 degrees of freedom and a robotized arm with 6 degrees of freedom for the prosthesis implantation visualized on the monitor with an avatar of the surgical instrument apt to manipulate the single prosthesis.

## Description

The present invention relates to a virtual platform conceived and developed for pre-surgery simulation and validation, in particular, for prosthesis surgery of the lumbo-sacral area of the human spine.

In the state of the art, there are known systems for pre-surgery planning of hip arthroprosthesis based on both TAC data and radiography, visualization software, recording and evaluation of the cardiological images and software for mapping the TAC values to finite elements models.

The technical problem solved by the present invention lies in the field of computerized simulations as a really innovative product, to ensure a reliable evaluation (as if the surgery is really carried out) of the results which can be obtained after prosthesis surgeries of the lumbo-sacral area of the human spine.

These and other advantages will be better highlighted in the following description of the invention which refers specifically to table 1-3, figures 1 to 5, in which it is represented a nonlimiting, preferred embodiment of the present invention. In particular:
Fig. 1 shows the scheme comprising the module for the virtual pre-surgery simulation and the module for the bio-mechanic validation of the simulated surgery;
Fig. 2 shows an example of simulation of a surgery for the implantation of a "Coflex" type prosthesis;
Fig. 3 shows the bi-manual interaction of the user with the 3D virtual environment;
Fig. 4 shows a visualization example of the stress at the intervertebral discs in terms of colour map;
Fig. 5 shows the flowchart of the macro-steps of the procedure using the platform.

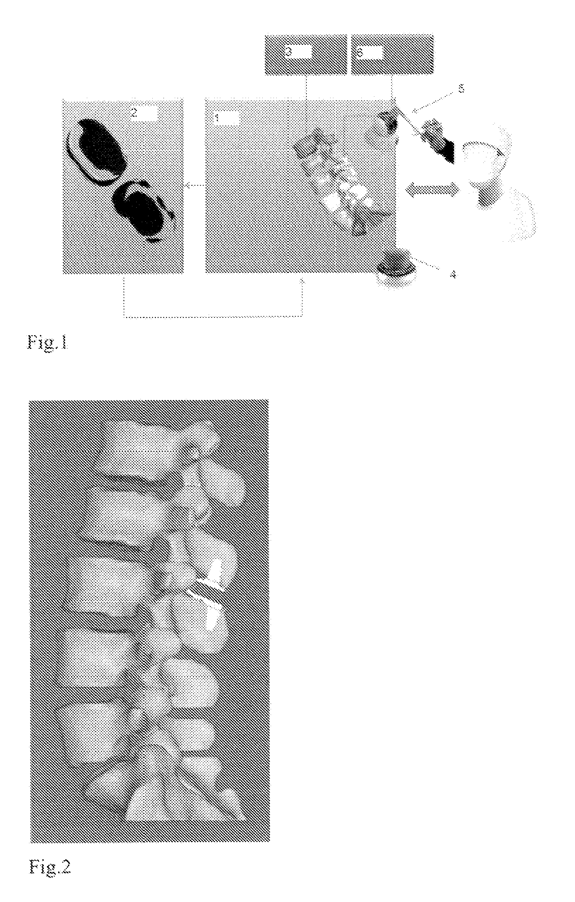

Referring to said figures, the platform object of the present invention consists of two modules (fig. 1): a module for virtual pre-surgery simulation of a prosthesis surgery (1) of the lumbosacral area of the human spine, and a module for the bio-mechanic validation of the simulated surgery (2), based on techniques of analysis to the finite elements (FEA-Finite Element Analysis).

The virtual prosthesis module (1) uses a metaphor of man-machine interaction which is identified by the acronym Medi-MUI (Medical Multimodal User Interface). It allows the doctor to simulate virtually a prosthesis surgery of the lumbosacral area of the spine of the specific patient interested, upon extraction of the 3D model (3) of the vertebral bodies interested by means of a TAC (Computerized Axial Tomography). These data are imported in the module in block form according to a well diffused standard ".stl".

In particular, the virtual prosthesis module is made up of a user graphic interface (GUI-Graphic User Interface) to visualize the anatomic area interested, provided with suitable library containing CAD reproductions (Computer-Aided Design) of the most common prosthesis (6) used in the surgery; interspinal prosthesis for discal assistance, as for example Cofles "U" (Paradigm Spine), Viking (Sintea Biotech), X-Stop (St. Francis Medical Technologies), Bacjac (Pioneer Surgical Technologies), as well as rigid prosthesis with vertebral coupling rods; of a navigation device (pan, rotate, zoom; 4) in 3D environment with 6 degrees of freedom; of a robotized arm with 6 degrees of freedom (5) for the prosthesis implantation, visualized on the monitor with an avatar of the surgical instrument apt to manipulate the single prosthesis.

Specifically, the user (medical-surgical personnel) can load and visualize the 3D model of the lumbosacral spine extracted by the TAC; choose the type and the dimensions of the prosthesis which is better indicated for the case of interest from the suitable database of prosthesis devices integrated in the platform; select one or more couples of vertebrae to be prosthesized and simulate the movement (opening or sliding) of the couple of vertebrae between which the prosthesis device has to be implanted, as it occurs in reality, when the surgeon repositions manually the vertebrae before introducing the prosthesis.

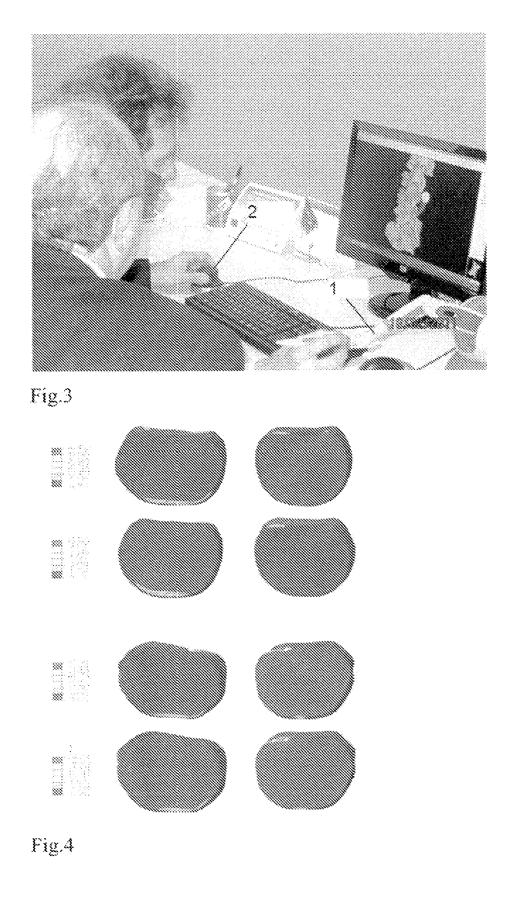

Moreover it is possible to interact (fig. 3) with the virtual environment in bi-manual mode; with the main hand, the surgeon controls the robotized arm (1) which simulates the surgical instrument to move the prosthesis and with the other hand, the surgeon controls the navigating device (pan, rotate, zoom; 2) of the scene, that is the spine (the interface is intuitive and eliminates the natural aversion of the surgeon to operate with mouse and keyboard). By using the arm, the prosthesis, selected in the database with drag&drop modes, can be manually introduced and positioned in the 3D scene.

Moreover the system allows to provide a force feedback to the doctor to simulate the interference of the prosthesis with the anatomic structures (bone, soft tissue, etc...) and to verify the correct geometric coupling of the prosthesis with the vertebral bodies.

Finally, it is possible to verify the good quality of the choice, evaluating possibly different prostheses, which will substitute the previous one chosen by the surgeon, maintaining the position and orientation thereof with respect to the spine.

The second module allows to realize the models for the analysis to the finite elements (FEA) both for the pre-surgery case and for the prothesized one (or ones). The FEA analysis aims at defining which, among the simulated prosthesis surgeries, has better dynamically responded in terms of residual functionality of the interested spine tract. Such analysis is carried out taking in consideration all the functional obligations linked to the anatomy of the interested area: intervertebral joint, ligaments, etc.... In particular, for each model four different analysis are carried out, from which it is obtained: an estimation of the relative rotation angle between the couples of vertebrae under bending, extension and lateral rotation load, and an analysis of the stresses of the intervertebral discs in the same above-described movements.

The comparison between the pre-surgery case and the prosthesized model can be made usable for the surgeon according to the animation mode of the functionality of the anatomic area or to the colour scale mapping of the stress of the intervertebral discs (fig. 4).

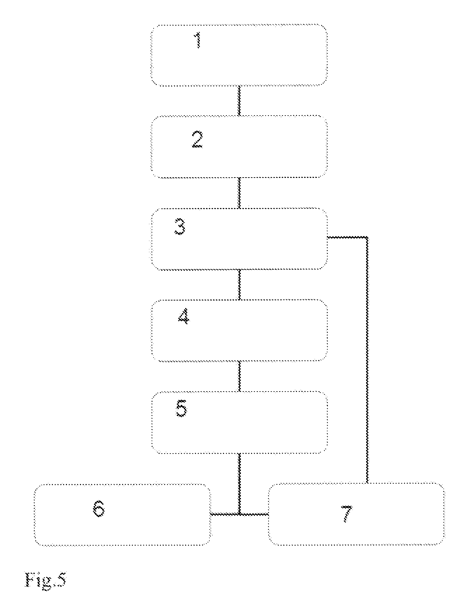

The protocol of usage of the platform provides different steps (fig. 5): preparatory steps such as for example the acquisition of the TAC scanning of the lumbosacral spine of the patient interested according to the DICOM standard (1) and the extraction and exportation of the 3D model of the lumbosacral area in .stl format (2); steps peculiar to the inventive object such as for example the pre-surgery simulation of the prosthesis surgery by means of GUI (3) and the analysis to the finite elements (FEM) of the residual functionality of the prosthesized model (4); clinic validation steps such as for example the comparison respect to the non-prosthesized case (5) which can respond positively (6) or negatively (7) to the surgery. In case of negative reaction, it is possible to choose a different prosthesis and to come back to the preparatory simulation step to obtain new results on the same subject.

The principle advantages of the present invention lie in a greater certainty on the predictability of the result expected for a certain choice of prosthesis, since the whole process starts from the 3D model of the spine of the patient to be subjected to the surgery; moreover, the analysis to the finite elements is set on morpho-metrical data obtained in vivo. This allows the surgeon to evaluate previously if the chosen prosthesis is apt for recovering the biomechanics of the spine of the patient interested.

Other advantages are the reduction of the surgery duration and of the anesthesia as well: when the surgeon enters the operatory room, he knows yet which prosthesis is to be implanted; and the elimination of possible medical-legal disputes connected to the success of the surgery.

By means of this system, it is moreover possible to realize previously custom prostheses, which are perfectly adaptable to the anatomy of the patient, thanks to the possibility to extend the database of the prosthesis devices.

## Claims

1. Virtual platform **characterized by** two modules: a first module for the virtual pre-surgery simulation of a prosthesis surgery of the lumbosacral area of the human spine and a second module for the bio-mechanic validation of the simulated surgery, based on techniques of analysis to the finite elements.

2. Platform according to claim 1, wherein said first module comprises:
- an user graphic interface for the visualization of the anatomic area interested;
- a device for navigating in 3D environment with 6 degrees of freedom;
- a robotized arm with 6 degrees of freedom for the prosthesis implantation, visualized on the monitor with an avatar of the surgical instrument apt to manipulate the single prosthesis.

3. Platform according to claim 2 wherein the user graphic interface is provided with suitable library of the most common prostheses used in the surgery; interspinal prostheses of discal assistance and rigid prostheses with vertebral coupling rods.

4. Platform according to claim 2 wherein the user interacts with the virtual environment in bi-manual way: with the main hand he controls the robotized arm which simulates the surgical instrument to move the prosthesis and with the other hand he controls the navigating device of the scene, that is the spine.

5. Platform according to claim 1 wherein said analysis to the finite elements is set on morpho-metrical data obtained *in vivo.*

6. Platform according to any one of claims 1 to 5 **characterized by** four different analysis for each model, from which it is obtained an estimation of the relative rotation angle between the couples of vertebrae and an analysis of the stresses of the intervertebral discs under bending, extension and lateral rotation load.

7. Protocol of usage of the platform **characterized by** the following steps:
- acquisition of the TAC scanning of the lumbosacral spine of the patient interested according to the DICOM standard
- extraction and exportation of the 3D model of the lumbosacral area in .stl format
- pre-surgery simulation of the prosthesis surgery by means of GUI
- analysis to the finite elements of the residual functionality of the prosthesized model
- comparison with respect to the non-prosthesized case.

8. Protocol according to claim 7 wherein said comparison between the pre-surgery case and the prosthesized model can be made usable for the surgeon with an animation mode of the functionality of the anatomic area or with a colour scale mapping of the stress of the intervertebral discs.
